**Europäisches Patentamt**

**European Patent Office**

**Office européen des brevets**

(19)

(11) Publication number: **0 203 708 B1**

# EUROPEAN PATENT SPECIFICATION

(12)

(45) Date of publication of patent specification: **12.06.91**

(51) Int. Cl.5: **C12N 1/04**, A01N 63/00, A01C 1/06, C05F 11/08

(21) Application number: **86303065.6**

(22) Date of filing: **23.04.86**

(54) Bacterial agricultural inoculants.

(30) Priority: **25.04.85 US 727029**

(43) Date of publication of application:
**03.12.86 Bulletin 86/49**

(45) Publication of the grant of the patent:
**12.06.91 Bulletin 91/24**

(84) Designated Contracting States:
**AT DE FR GB IT NL SE**

(56) References cited:
**EP-A- 0 017 565**
**DE-A- 3 335 643**
**FR-A- 2 501 716**
**US-A- 3 168 796**
**US-A- 3 420 933**

**CHEMICAL ABSTRACTS, vol. 73, no. 15, 12 October 1970, page 290, abstract no. 76312f, Columbus, Ohio, US**

(73) Proprietor: **AGRACETUS**
**8520 University Green**
**Middleton Wisconsin 53562(US)**

(72) Inventor: **Paau, Alan**
**5405 Jonquil Court**
**Middleton Wisconsin 53562(US)**

(74) Representative: **Bentham, Stephen et al**
**J.A. Kemp & Co. 14 South Square Gray's Inn**
**London WC1R 5EU(GB)**

Rank Xerox (UK) Business Services

## Description

The present invention relates to bacterial agricultural products, including Rhizobium inoculants, and methods for making and preserving such agricultural products.

It is well known that leguminous plants under certain conditions "fix nitrogen" directly from the air and convert it to organic nitrogenous compounds, thereby providing nitrogen to the plant for protein synthesis and also enriching the soil around the leguminous plants by leaving nitrogenous nutrients in the soil for later crops. In actual fact, of course, the plants themselves do not fix nitrogen, but the nitrogen fixation occurs in Rhizobium bacteria which exist symbiotically with the legumes in nodules formed in the roots of the plants. Examples of leguminous plants which are capable of symbiotic relationship with Rhizobium bacteria are peas, beans, alfalfa, red clover, white clover, vetch, lupines, and the like.

Other agriculturally important non-leguminous plants, such as grasses and grains, are unable to fix nitrogen directly from the air. Non-Leguminous plants generally depend entirely upon combined nitrogen in the soil, such as nitrates and ammonium salts for nitrogen for protein synthesis. After a series of such crops have been grown on any given field, the combined nitrogen in the soil becomes depleted. Consequently, crop rotation is often practiced, whereby nitrogen-fixing plants are grown in rotation with non-nitrogen-fixing plants to replenish soil nitrogen. Furthermore, some legumes, particularly soybeans, peas, beans and alfalfa, are commercially important as crops themselves, and the growth of these crop plants is greatly facilitated by ample combined nitrogen availability.

The particular Rhizobium bacteria necessary to a given nitrogen-fixing plant may not be universally present in the soil. Different Rhizobium species are adapted to form nodules only in legumes of specific species. Therefore, it is a quite common practice to inoculate the seeds of leguminous plants with an appropriate culture of Rhizobium bacteria. The inoculation can be done by coating the seeds, dusting planted seeds or crops, or by spreading inoculant in the furrows of planted leguminous seeds.

One method of inoculating leguminous seeds is to maintain the Rhizobium bacteria culture in an active living state, by mixing a moist culture of the bacteria with a carrier such as humus or peat. The carrier maintains the bacteria in a moist, living state. However, the shelf life of such live bacterial cultures can be relatively short, because the bacteria die under conditions of storage due to the relative shortage of food and moisture in their environment. An example of a moist type of inoculant mixture is described in U.S. Patent No. 2,726,948 to Erickson, wherein an active moist bacterial culture is mixed with a mixture of peat, charcoal and limestone, so that water is present to the level of approximately 38% by weight of the whole.

Another method of preparing legume seed inoculants is to convert the bacteria culture to a state of dormancy. One known method used to create dormant bacteria is freeze-drying, as described in U.S. Patent No. 3,168,796 to Scott, et al. In Scott, Rhizobium bacteria are freeze-dried at temperatures in the range of -35 to -70°C. The dried bacterial cake is then ground and blended with a powdered carrier, which carrier is a non-hydroscopic inert powder less than 40 microns in size. The weight ratio of bacteria to carrier in the Scott patent is in the range of 5 to 400 milligrams bacteria per ounce of carrier. According to the Scott patent, it is critical that water be excluded during the mixing step between bacteria and carrier.

Although freeze-drying Rhizobium bacteria prior to mixing with a carrier usually gives a high initial recovery, the bacteria do not always remain stable for long storage periods. Therefore, it would be advantageous to provide a dry, dormant Rhizobium inoculant which can be easily prepared and which will be stable over fairly long storage times with high yields of viable Rhizobium upon re-exposure to moisture.

It is known from EP 17565A to incorporate a bacteria such as Rhizobium into a polymeric, preferably polysaccharide, gel. The gel mixture may then be dried by the additional step of adding a powder such as silica. It has now been found that good results may be obtained without the need for the polysaccharide gel.

Some have suggested that other, non-Rhizobium, bacteria may be beneficial to some crop plants. Many terrestrial bacterial species are known and it is quite possible that some may be particularly helpful in the cultivation of field crops because of symbiotic relationships formed between plant and bacteria. In the event such associations are identified, it would become necessary to be able to effectively deliver the bacteria to the field in order to take advantage of this symbiosis.

According to the present invention, there is provided a method for making an agriculturally useful inoculant of dormant bacteria, characterised in that it comprises only the steps of:

(a) maintaining a concentrated suspension of bacteria, substantially separated from its culture medium at a temperature in the range of about 0 - 30°C for a period in the range of about 0 - 96 hours under aseptic conditions;

(b) mixing the bacterial suspension with a porous granular perlite or charcoal carrier such that the weight ratio of dry carrier to concentrated bacterial suspension is in the range of about 0.5 to 1.5; and

(c) air drying the bacteria-carrier mixture for a period of about 2 - 10 days under aseptic conditions, preferably at a temperature in the range 22 - 30°C.

The dormant bacteria may be either Rhizobium or any other useful species.

It is an object of the present invention to provide a bacterial inoculant in which the bacteria will be dormant and easily handleable, yet in which the bacteria will be stable and viable during extended storage time.

It is a further object of the present invention to provide a seed coating for legumes which includes a Rhizobium inoculant in which the coating and the seed will be stable during long storage periods.

The present invention discloses a method for making an agriculturally useful composition including a viable bacterial component, especially useful with Rhizobium bacteria. The bacteria culture itself can be efficiently grown either on a semisolid support such as AMA-agar or in the liquid state (AMA-liquid). After a suitable bulk quantity of the appropriate desired strain of Rhizobium or other bacteria has been cultured, the bacteria are harvested from the culture medium. Suitable Rhizobium species include R. japonicum, used with soybeans; R. trifolii, used with clovers; R. meliloti, used with alfalfa and sweet clovers; R. leguminosarum, used with peas and vetches; R. phascoli, used with garden beans; and R. lupini, used with lupines. Mixtures of the separate species of Rhizobium may also be used. The harvesting method selected depends on the type of culture used. For instance, if a semisolid support such as AMA-agar is used, the bacteria can be harvested by scraping the culture off the top of the agar. If a liquid culture is used, the bacteria can be separated from its culture medium by centrifugation.

Once the Rhizobium bacteria have been substantially separated from the culture medium, the bacteria are maintained at a temperature in the range of about 0 to 30°C for a period in the range of about 0 to 96 hours under aseptic conditions. Preferably, the bacterial suspension is maintained at a temperature in the range of about 22 to 30°C, i.e. a normal room temperature, for a period in the range of about 3 to 25 hours under aseptic conditions, followed by further maintaining the bacterial suspension at a temperature in the range of about 0 to 15°C for a period in the range of about 3 to 20 hours under aseptic conditions. The bacteria are maintained as a concentrated liquid suspension during this step. At this stage, after the bulk of the liquid medium has been removed, the bacteria are no longer actively growing. For Rhizobium, the density should be on the order of $10^7$ to $10^{12}$ bacteria per milliliter. The concentrated bacteria suspension should be a dense liquid appearing more like a viscous paste.

The concentrated bacterial suspension is then mixed with a porous, chemically inert granular carrier such that the weight ratio of concentrated bacteria suspension to dry carrier is in the range of about 0.5 to 1.5, preferably about 1.0. Examples of suitable carriers include vermiculite, perlite, and charcoal. If charcoal is used, a saturated saccharide solution, preferably selected from the group consisting of sucrose, lactose, trehalose, sorbitol and adonital, is added to the bacterial suspension prior to the step of maintaining the bacteria at 0 to 30°C. The weight of the saturated saccharide solution added to the bacteria suspension is in a ratio in the range of about 0.5 - 1.5, preferably about 1.0 by weight. Charcoal carriers are advantageous and may be preferable for some applications because the charcoal may absorb Rhizobium-toxic compounds, such as alkaloids and lignin compounds, which are naturally released by many legume seeds during germination.

Next, the bacteria-carrier mixture is slowly dried, preferably in air, at about room temperature, i.e. 22 to 30°C, for a period of about 2 to 10 days under aseptic conditions. The exact time period will, of course, vary depending on the concentration of bacteria in the liquid culture. The drying should continue until the bacteria-carrier mixture appears totally dried. The remaining moisture content of the dried mixture should approximate the relative humidity of the environment.

The composition resulting after the completion of drying includes dried, dormant yet viable Rhizobium together with the porous granular carrier in a loose cake. The cake is readily friable to yield a clumped, coarsely granular product. This granular product may or may not be re-ground to a more powdery form depending on the particular delivery strategy to be used.

Thus, depending on the delivery procedure to be used, the bacteria-carrier mixture can then be ground to a relatively fine powder for use in dusting or coating directly on legume seeds with or without an adhesive or can be dusted on the field before or during planting. Alternatively coarser granuler bacteria-carrier mixture might not be ground, and then the dried granular mixture can be easily and directly introduced into the furrows during planting to inoculate the seeds. Thus the material produced from the drying of the bacteria-carrier mixture is readily adaptable to various delivery methods as may be desired for any given application.

A Rhizobium-inoculated leguminous seed coating prepared by the present invention would include a mixture of Rhizobium bacteria together with a porous, chemically inert carrier, wherein the bacteria-carrier mixture has been air-dried to a substantially moisture free state. The weight ratio of concentrated bacteria culture to dry carrier should be in the range of about 0.5 to 1.5, preferably about 1.0. Any of the Rhizobium species mentioned above can be used in this leguminous seed coating. The preferable carrier is selected from the group consisting of vermiculite, perlite, and charcoal. When charcoal is selected, the leguminous seed coating further includes a saccharide. The saccharide is introduced in a saturated solution to the bacterial suspension at a weight ratio of saccharide solution to bacterial suspension in the range of about 0.5 - 1.5, preferably about 1.0. Examples of suitable saccharides include sucrose, lactose, trehalose, sorbitol and adonital. It may be preferable to grind the dried bacteria-carrier mixture prior to coating the leguminous seed. If so, such grinding is preferably done after the mixture has been air-dried.

While this method is particularly useful for Rhizobium species for which presently recognized agriculture uses exist, it has also been found useful in drying viable cultures of other non-Rhizobium species, whether for agricultural or other applications.

The following non-limitative examples are intended to illustrate the present invention.

Example 1

The bacteria used in this Example was a strain of Rhizobium japonicum, isolated from a soybean field in Louisiana. In each of the five experiments comprising Example 1, the bacteria were cultured for 14 days on AMA-agar. After culturing, the bacteria were harvested by scraping the culture from the agar support. The two carriers tested in this Example were perlite and charcoal. When perlite was used, the bacteria were first maintained at two different temperatures ($T_1$ and $T_2$) for a varying number of hours, and then mixed with the perlite carrier. When charcoal was selected as the carrier, the bacteria were first mixed with an equal weight of saturated sucrose and then maintained at the different temperatures ($T_1$ and $T_2$) for a varying number of hours. After the bacteria had been maintained for the required period of time, the bacteria were then mixed with an equal weight of charcoal. Table 1 illustrates the conditions for the five experiments which comprise Example 1. Table 2 contains the results of the experiment of Example 1, expressed as viable Rhizobium per gram dried product. The results illustrate the stability which can be achieved with the agricultural product of the present invention.

## Table 1: Conditions of Example 1

| Experiment | culture | weight ratio, carrier/ bacteria | weight ratio, saccharide/ bacteria | hours at $T_1$ | hours at $T_2$ |
|---|---|---|---|---|---|
| (a) | 14 days, AMA-agar | 0.5 perlite | – | 4 hr, 23°C | 18 hr, 10°C |
| (b) | 14 days, AMA-agar | 1.0 perlite | – | 4 hr, 23°C | 18 hr, 10°C |
| (c) | 14 days, AMA-agar | 1.0 perlite | – | 18 hr, 23°C | 7 hr, 10°C |
| (d) | 14 days, AMA-agar | 1.0 charcoal | 1.0 sucrose | 4 hr, 23°C | 18 hr, 10°C |
| (e) | 14 days, AMA-agar | 1.0 charcoal | 1.0 sucrose | 18 hr, 23°C | 7 hr, 10°C |

### Table 2: Results of Example 1

**(viable Rhizobium per gram dried product)**

| Experiment | 1 week | 11 weeks | 13 weeks | 43 weeks | 48 weeks |
|---|---|---|---|---|---|
| (a) | $10^9$ | $10^9$ | | | $10^7$ |
| (b) | $10^9$ | $10^8$ | | | $10^8$ |
| (c) | $10^9$ | $10^9$ | $10^8$ | | |
| (d) | $10^9$ | $10^8$ | | | $10^6$ |
| (e) | $10^9$ | $10^9$ | $10^9$ | $10^8$ | |

Example 2

In this Example, Rhizobium japonicum, isolated from a soybean field in Illinois, was tested. In the four experiments which comprise this Example, two different culture methods were used. In the first two experiments, the bacteria were cultured for 14 days on AMA-agar. In the second two experiments of this Example, the bacteria were cultured for 7 days on AMA-agar. As in Example 1, following harvesting of the bacteria, the bacteria was maintained at different temperatures for different periods of time prior to mixing with a carrier. Also as in Example 1, when charcoal was the carrier, the bacteria were first mixed with an equivalent weight of a saturated sucrose solution prior to maintaining the bacteria at $T_1$. The conditions used in Example 2 are illustrated in Table 3. The results of this Example are given in Table 4.

## Table 3:  Conditions of Example 2

| Experiment | culture | weight ratio, carrier/ bacteria | weight ratio, saccharide/ bacteria | hours at $T_1$ | hours at $T_2$ |
|---|---|---|---|---|---|
| (a) | 14 days, agar | 1.0 charcoal | 1.0 sucrose | 4 hr, 23°C | 18 hr, 10°C |
| (b) | 14 days, agar | 0.5 perlite | - | 4 hr, 23°C | 18 hr, 10°C |
| (c) | 7 days, agar | 1.0 perlite | - | 4 hr, 23°C | 18 hr, 10°C |
| (d) | 7 days, agar | 1.0 perlite | | 22 hr, 23°C | - |

## Table 4:  Results of Example 2

### (viable Rhizobium per gram dried product)

| Experiment | 1 week | 5 weeks | 11 weeks | 15 weeks | 48 weeks |
|---|---|---|---|---|---|
| (a) | $10^7$ | | $10^7$ | | |
| (b) | $10^{10}$ | | $10^{10}$ | | $10^6$ |
| (c) | $10^9$ | $5 \times 10^8$ | | | |
| (d) | $10^9$ | $5 \times 10^8$ | | $2 \times 10^8$ | |

Example 3

In this Example, a Rhizobium japonicum culture isolated from a field in Iowa was tested. The conditions of Example 3 are given in Table 5, and the results of Example 3 expressed in viable Rhizobium per gram dried product are given in Table 6.

Table 5:   Conditions of Example 3

| Experiment | culture | weight ratio, carrier/ bacteria | weight ratio, saccharide/ bacteria | hours at $T_1$ | hours at $T_2$ |
|---|---|---|---|---|---|
| (a) | 14 days, agar | 1.0 perlite | – | 4 hr, 23°C | 18 hr, 10°C |
| (b) | 14 days, agar | 1.0 charcoal | 1.0 sucrose | 4 hr, 23°C | 18 hr, 10°C |
| (c) | 7 days, agar | 1.0 perlite | – | 24 hr, 23°C | – |

Table 6:   Results of Example 3

(viable Rhizobium per gram dried product)

| Experiment | 1 week | 5 weeks | 6 weeks | 9 weeks | 14 weeks | 18 weeks | 19 weeks |
|---|---|---|---|---|---|---|---|
| (a) | $10^{10}$ | $10^{10}$ | | | $10^9$ | | $10^7$ |
| (b) | $10^{10}$ | $10^8$ | | $10^6$ | | | |
| (c) | $10^{10}$ | | | $10^{10}$ | | $10^9$ | $10^7$ |

## Example 4

In this example, another Rhizobium japonicum isolated from Louisiana was tested. The conditions of Example 4 are given in Table 7, and the results of this Example are given in Table 8.

Table 7:   Conditions of Example 4

| Experiment | culture | weight ratio, carrier/ bacteria | weight ratio, saccharide/ bacteria | hours at $T_1$ | hours at $T_2$ |
|---|---|---|---|---|---|
| (a) | 14 days, agar | 1.0 perlite | – | 4 hr, 23°C | 18 hr, 10°C |
| (b) | 14 days, agar | 1.0 charcoal | 1.0 sucrose | 4 hr, 23°C | 18 hr, 10°C |

### Table 8:  Results of Example 4

#### (viable Rhizobium per gram dried product)

| Experiment | 1 week | 5 weeks | 9 weeks | 14 weeks | 19 weeks |
|---|---|---|---|---|---|
| (a) | $10^{10}$ | | $10^{10}$ | $10^8$ | $10^7$ |
| (b) | $10^{10}$ | $10^9$ | $10^9$ | $10^8$ | $10^7$ |

Example 5

In this example, another Rhizobium japonicum isolate from Iowa was utilized. The conditions of Example 5 are expressed in Table 9. The results of this Example are given in Table 10.

### Table 9:  Conditions of Example 5

| Experiment | culture | weight ratio, carrier/ bacteria | weight ratio, saccharide/ bacteria | hours at $T_1$ | hours at $T_2$ |
|---|---|---|---|---|---|
| (a) | 14 days, agar | 1.0 perlite | – | 4 hr, 23°C | 18 hr, 10°C |
| (b) | 14 days, agar | 1.0 charcoal | 1.0 sucrose | 4 hr, 23°C | 18 hr, 10°C |

### Table 10:  Results of Example 5

#### (viable Rhizobium per gram dried product)

| Experiment | 1 week | 5 weeks | 9 weeks | 14 weeks | 19 weeks |
|---|---|---|---|---|---|
| (a) | $10^{10}$ | $10^{10}$ | $10^9$ | $10^8$ | $10^7$ |
| (b) | $10^8$ | $10^8$ | $10^7$ | | |

Example 6

In this example, the same method was tested on two non-Rhizobium bacteria. Two bacterial cultures, designated LAlc-2 and LAlc-6, were isolated from the roots of soybean plants in the state of Louisiana. Neither culture will nodulate a legume, as does Rhizobium. Both cultures are bacillus shaped, LAlc-2 being a long bacillus while LAlc-6 is short, while Rhizobium is, of course, a short rod shape. Both cultures are gram positive (Rhizobium is gram negative) and both cultures grow well on nutrient broths and agars which do not favor Rhizobium cultures.

Using perlite as the granular carrier, dried mixtures of each of these cultures was prepared. The dried preparations were examined and found to contain $1.2 \times 10^9$ viable bacteria per gram for LAlc-2 and $1.9 \times 10^9$ viable bacteria per gram for LAlc-6. In each case approximately 5% of the bacteria were revived.

It is to be understood that modification of the above-described method for making an agriculturally useful bacterial composition and the agricultural product described here is possible within the present invention. For example it may be advantageous to add other constituents to the dried bacterial product of the present invention. Other agricultural products, such as dry or liquid fertilizer or herbicides or pesticides not injurious to the bacteria could be added to the mixture. While it is particularly useful, in addition, to use

EP 0 203 708 B1

the dried bacterial composition disclosed here as a seed coating, it could also be applied in furrow, or re-wetted and sprayed, or applied in some other fashion.

## Claims

1. A method for making an agriculturally useful inoculant of dormant bacteria, characterised in that it comprises only the steps of:

   (a) maintaining a concentrated suspension of bacteria, substantially separated from its culture medium at a temperature in the range of about 0 - 30° C for a period in the range of about 0 - 96 hours under aseptic conditions;

   (b) mixing the bacterial suspension with a porous granular perlite or charcoal carrier such that the weight ratio of dry carrier to concentrated bacterial suspension is in the range of about 0.5 to 1.5; and

   (c) air drying the bacteria-carrier mixture for a period of about 2 - 10 days under aseptic conditions, preferably at a temperature in the range 22 - 30° C.

2. A method according to claim 1, characterised in that step (a) is initially carried out at a temperature of 22 - 30° C for a period of 3 - 25 hours, followed by maintenance at a temperature in the range 0 - 15° C for a period of 3 - 20 hours.

3. A method according to either claim 1 or claim 2, characterised in that the bacteria are Rhizobium.

4. A method according to any one of claims 1 to 3, characterised in that the carrier is perlite.

5. A method according to any one of claims 1 to 3, characterised in that the carrier is charcoal, and by the step of adding a saturated saccharide solution to the bacteria, prior to the step of maintaining the bacteria at 0 - 30° C, such that the weight ratio of the solution to bacterial suspension is in the range of about 0.5 - 1.5.

6. A method according to claim 5, characterised in that the saccharide in the saccharide solution comprises sucrose, lactose, trehalose, sorbitol or adonital.

7. A method according to any one of the preceding claims, characterised by the further step of grinding the bacteria-carrier mixture to a powder.

8. An inoculant for legume seeds, consisting only of a mixture of dry, dormant but viable Rhizobium bacteria together with a porous, perlite or charcoal carrier, characterised in that the bacteria-carrier mixture has been air-dried tc a substantially moisture-free state and the weight ratio, pre-drying, of concentrated bacteria suspension to dry carrier is in the range of about 0.5 - 1.5.

9. An agriculturally useful composition consisting only of legume seeds coated with a coating of dried, dormant but viable Rhizobium bacteria mixed with a porous, perlite or charcoal carrier, wherein the bacteria-carrier mixture has been air-dried to a substantially moisture-free state and the weight ratio, pre-drying, of concentrated bacteria suspension to dry carrier is in the range of about 0.5 to 1.5.

## Revendications

1. Procédé de préparation d'un inoculum de bactéries dormantes utile en agriculture, caractérisé en ce qu'il comprend seulement les étapes consistant à:

   (a) maintenir une suspension concentrée de bactéries, essentiellement séparée de son milieu de culture, à une température située dans un intervalle d'environ 0-30° C pendant une durée d'environ 0-96 heures dans des conditions aseptiques;

   (b) mélanger la suspension bactérienne avec un support granulaire poreux de perlite ou de charbon de bois de manière que le rapport pondéral du support sec à la suspension bactérienne concentrée soit dans un intervalle d'environ 0,5 à 1,5; et

9

(c) sécher à l'air le mélange bactéries-support pendant une durée d'environ 2-10 jours dans des conditions aseptiques, de préférence à une température située dans un intervalle de 22-30°C.

2. Procédé selon la revendication 1, caractérisé en ce que l'étape (a) est initialement conduite à une température de 22-30°C pendant une période de 3-25 heures, suivie par un maintien à une température située dans un intervalle de 0-15°C pendant une période de 3-20 heures.

3. Procédé selon l'une des revendication 1 ou 2, caractérisé en ce que les bactéries sont des Rhizobium.

4. Procédé selon l'une quelconque des revendications 1 à 3, caractérisé en ce que le support est la perlite.

5. Procédé selon l'une quelconque des revendications 1 à 3, caractérisé en ce que le support est le charbon de bois, et par l'étape d'addition d'une solution saturée de saccharide aux bactéries, avant l'étape de maintien des bactéries à 0-30°C, de manière que le rapport pondéral de la solution à la suspension bactérienne soit dans un intervalle d'environ 0,5-1,5.

6. Procédé selon la revendication 5, caractérisé en ce que le saccharide dans la solution de saccharide comprend du saccharose, du lactose, du tréhalose, du sorbitol ou de l'adonital.

7. Procédé selon l'une quelconque des revendications précédentes, caractérisé par l'étape supplémentaire de broyage du mélange bactéries-support en une poudre.

8. Inoculum pour semences de légumes, constitué uniquement d'un mélange de bactéries Rhizobium sèches, dormantes mais viables avec un support poreux de perlite ou de charbon de bois, caractérisé en ce que le mélange bactéries-support a été séché à l'air jusqu'à un état essentiellement dépourvu d'humidité, et en ce que le rapport pondéral avant séchage de la suspension de bactéries concentrées au support sec est dans un intervalle d'environ 0,5-1,5.

9. Composition utile en agriculture constituée uniquement de semences de légumes revêtues d'un revêtement de bactéries Rhizobium séchées, dormantes mais viables, mélangées à un support poreux de perlite ou de charbon de bois, où le mélange bactéries-support a été séché à l'air jusqu'à un état essentiellement dépourvu d'humidité et où le rapport pondéral, avant séchage, de la suspension de bactéries concentrées au support sec est dans un intervalle d'environ 0,5 à 1,5.

## Ansprüche

1. Verfahren zur Herstellung eines landwirtschaftlich brauchbaren Inokulans aus ruhenden Bakterien, dadurch gekennzeichnet, daß es nur die Stufe umfaßt:
(a) Aufbewahren einer konzentrierten Suspension von Bakterien, die im wesentlichen von ihrem Kulturmedium abgetrennt sind, bei einer Temperatur im Bereich von ca. 0 bis 30°C während einer Zeit im Bereich von ca. 0 bis 96 Stunden unter aseptischen Bedingungen;
(b) Mischen der Bakteriensuspension mit einem porösen körnigen Perlit- oder Holzkohlen-Träger in einer solchen Weise, daß das Gewichtsverhältnis des trockenen Trägers zu der konzentrierten Bakteriensuspension im Bereich von ca. 0.5 bis 1.5 liegt; und
(c) Lufttrocknen der Bakterien-Träger-Mischung während eines Zeitraums von ca. 2 bis 10 Tagen unter aseptischen Bedingungen, vorzugsweise bei einer Temperatur im Bereich von 22 - 30°C.

2. Verfahren nach Anspruch 1, dadurch gekennzeichnet, daß Stufe (a) zunächst bei einer Temperatur von 22 bis 30°C während einer Zeit von 3 bis 25 Stunden durchgeführt wird, gefolgt von einem Aufbewahren bei einer Temperatur im Bereich von 0 bis 15°C während einer Zeit von 3 bis 20 Stunden.

3. Verfahren nach Anspruch 1 oder 2, dadurch gekennzeichnet, daß die Bakterien Rhizobium sind.

4. Verfahren nach einem der Ansprüche 1 bis 3, dadurch gekennzeichnet, daß der Träger Perlit ist.

5. Verfahren nach einem der Ansprüche 1 bis 3, dadurch gekennzeichnet, daß der Träger Holzkohle ist, und durch die Stufe, in der man vor der Stufe des Aufbewahrens der Bakterien bei 0 bis 30° C eine gesättigte Saccharid-Lösung zu den Bakterien so zugibt, daß das Gewichtsverhältnis der Lösung zu der Bakteriensuspension im Bereich von ca. 0.5 bis 1.5 liegt.

6. Verfahren nach Anspruch 5, dadurch gekennzeichnet, daß das Saccharid in der Saccharid-Lösung Sucrose, Lactose, Trehalose, Sorbit oder Adonit umfaßt.

7. Verfahren nach einem der vorhergehenden Ansprüche gekennzeichnet durch die weitere Stufe des Mahlens der Bakterien-Träger-Mischung zu einem Pulver.

8. Inokulans für Leguminosen-Samen, das nur aus einer Mischung von trockenen, ruhenden aber lebensfähigen Rizobiumbakterien mit einem porösen Perlit- oder Holzkohle-Träger besteht, dadurch gekennzeichnet, daß die Bakterien-Träger-Mischung auf einen im wesentlichen feuchtigkeitsfreien Zustand luftgetrocknet wurde und das Gewichtsverhältnis, vor der Trocknung, der konzentrierten Bakteriensuspension zu dem trockenen Träger im Bereich von ca. 0.5 bis 1.5 liegt.

9. Landwirtschaftlich brauchbare Zusammensetzung, die nur aus Leguminosen-Samen besteht, die mit einem Überzug aus einer Mischung getrockneter, ruhender aber lebensfähiger Rhizobium-Bakterien mit einem porösen Perlit- oder Holzkohle- Träger beschichtet sind, wobei die Bakterien-Träger-Mischung auf einen im wesentlichen feuchtigkeitsfreien Zustand luftgetrocknet wurde und das Gewichtsverhältnis, vor der Trocknung, der konzentrierten Bakteriensuspension zu dem trockenen Träger im Bereich von ca. 0.5 bis 1.5 liegt.